# EUROPEAN PATENT APPLICATION

(11) **EP 4 621 049 A1**
(43) Date of publication of application: **24.09.2025**
(21) Application number: 24164530.8
(22) Date of filing: 19.03.2024
(51) Int. Cl.: C12N 7/00

(54) **MULTISPECIFIC PARTICLES**

(71) Applicant: Invitris GmbH, 81476 Munich (DE)
(72) Inventor: GROSSMANN, Patrick, 81476 München (DE); VOGELE, Kilian, 82399 Raisting (DE)
(74) Representative: Weickmann & Weickmann PartmbB

(57) **Abstract**

The present invention relates to a synthetic phage-like particle having specificity for at least two different target cells, compositions comprising such phage-like particles, methods for providing as well as their use in medicine, chemistry, biotechnology, agriculture and/or food industry.

## Description

The present invention relates to a synthetic phage-like particle having specificity for at least two different target cells, compositions comprising such phage-like particles, methods for providing as well as their use in medicine, chemistry, biotechnology, agriculture and/or food industry.

Infections caused by antibiotic-resistant bacteria pose a significant challenge to modern healthcare. The World Health Organization (WHO) mentions infections as one of the top threats to global health, food security, and development today. Even once-treatable infections become increasingly difficult to manage, leading to higher mortality rates, prolonged hospital stays, and escalated medical costs.

For the US alone, the Centers for Disease Control and Prevention reports that 2.8 million people acquire antibiotic-resistant infections annually, resulting in more than 35,000 deaths and costing the healthcare system upwards of $20 billion in direct expenses ("Antibiotic resistance threats in the United States 2019"). Europe faces a similar predicament, with antibiotic resistance causing approximately 33,000 deaths yearly and imposing a financial burden estimated at €1.5 billion on its healthcare systems. (A. Cassini et al; Lancet Vol.19.1; 2019).

According to the WHO, a coordinated global response is required to curb antibiotic-resistant pathogens, emphasizing the judicious use of antibiotics, the promotion of infection prevention and control, and the exploration and development of new antibiotics and alternative therapies such as phage therapy and/or antimicrobial peptides (AMPs) such as bacteriocins.

Alongside the development of new broad-spectrum antibiotics, phage-based therapy utilizing the natural characteristics of bacteriophages to specifically affect bacteria and kill them, usually by lysing the bacteria or by inhibiting their replication system, appears to have a great potential for the treatment of multi-resistant bacterial infections.

Bacteriophages (herein interchangeably also called "phages") are viruses that specifically infect a host bacterium and proliferate at the expense of this host. They are composed of proteins that encapsulate a DNA or RNA molecule. Within their lytic state, bacteriophages replicate within their host bacterium by injecting their viral genetic material into the host cell, thereby effectively taking over the cells functions for the production of progeny phages which, in the end, leads to the rupture of the cell wall and subsequent bacterial cell death.

Beyond their promising therapeutic potential, bacteriophages offer a wide range of biotechnological applications. These include evolution-based selection methods for improving enzymes and other proteins, phage display techniques for generating therapeutic antibodies, and applications in food safety, such as the detection and control of pathogenic bacteria during production and storage.

The use of bacteriophages to specifically target and eliminate pathogenic bacteria has long been recognized and is becoming increasingly relevant, particularly with the global rise in multidrug resistant bacterial strains, such as Methicillin-resistant Staphylococcus aureus (MRSA). These infections, often contracted in hospitals, are notoriously difficult to treat with conventional antibiotics, and the pace of developing new antibiotics lags far behind the spread of resistance.

The development of phage-based therapeutics and diagnostics, however, faces challenges related to the production and/or modification of bacteriophages. It has proven difficult to produce the quantities of bacteriophages or modified bacteriophages necessary for therapeutic use. For therapeutic applications, it would also be desirable, at least from a regulatory perspective, to rapidly expand the "host range" of bacteriophages with a tested and proven safety profile to include new pathogenic bacteria.

To address these needs the present invention discloses novel synthetic phages and phage-like particles that can be tailored to a broad or highly specific host range as required, as well as nucleic acids encoding these phage-like particles.

The application further discloses methods for the cell-free production of phages, phage-like particles and compositions comprising such phages or phage-like particles as well as their use in biotechnological applications in particular in the production of food and/or in medical applications, in particular in the diagnosis and treatment of infectious diseases in humans and animals.

### DETAILED DESCRIPTION

The recognition and attachment of a bacteriophage to its cognate host bacterium or host cell is a key step in the infection process, characterized by the specific interaction between the phage's tail fibers and receptors on the surface of the bacterial cell. The tail fibers of a bacteriophage are protein structures which are usually attached to the tail structure of the phage via a base plate. These fibers are highly specialized and rely on recognition mechanisms that specifically target molecular structures on the surface of the host bacterial cell. The receptors on the host cell may be, for example, lipopolysaccharides, teichoic acids, proteins, or other cell wall and/or membrane components.

The recognition of the surface structure on the target cell by binding site-specific proteins, which are located mainly at the distal end of the tail fibers is, in terms of specificity and affinity, comparable to specific high-affinity binding of an antibody to its epitope and is crucial as it determines which host cell a given phage can infect. This specificity allows phages to be used selectively against specific bacterial pathogens, leaving the remaining natural microflora largely unaffected.

Once the phage is attached to its host cell structural changes can occur in the phage tail and in the cell wall of the bacterium which is thereby usually perforated, allowing e.g. phage DNA and/or phage proteins to be transferred into the host cell.

Even though the transfer or translocation of phage DNA into the host cell, followed by phage production in the host and lysis of the host, represents the most common scenario, in some cases the perforation of the cell wall and/or cell membrane by tail-like or phage-like structures is sufficient to damage the host cell and to initialize killing.

In the latter case, therefore, neither the phage's envelope (also referred to as head or capsid) nor its genetic material is necessary to damage the host.

In the context of the invention, such an envelope-free or capsid-free phage-like particle is referred to as headless-phage. Headless-phages are usually modified or engineered - also known as synthetic - phage-like structures which are often based on one or more naturally occurring phages and which may comprise further modifications even of non-phage origin.

The specificity and suitability of the phage-like particle for specific host cell attachment, optionally comprising subsequent damage of the host cell, therefore, solely depends on the multi tail fiber system of the phage-like particle. The host cell specificity of the phage-like particle is therefore extended to further host cells by a multi-fiber system comprising tail fiber species with diverging specificity.

### Synthetic phage-like particles

A first aspect of the invention therefore relates to a synthetic phage-like particle having specificity for at least two different types of target cells,
wherein said synthetic phage-like particle comprises at least a functional tail comprising a baseplate and a multi tail fiber system,
which multi tail fiber system comprises at least two different tail fibers having specificity for different types of target cells,
wherein said tail fibers preferably emanate from said baseplate.

In one embodiment, the inventive synthetic phage-like particle comprises at least a functional tail comprising a baseplate and a multi tail fiber system comprising two different types of tail fibers, wherein
a first tail fiber type specifically binds to a first type of target cells and
a second tail fiber type specifically binds to a second type of target cells, and
wherein said tail fibers preferably emanate from said baseplate.

In general, the synthetic phage-like particles disclosed herein are preferably synthetic phage-tail like particles. Such phage-tail like particles are derived from a bacteriophage tail structure and/or are structurally and/or functionally similar to a bacteriophage tail structure. Such phage-tail like particles may be derived from bacteriophages, e.g. by removing the capsid structure or may be of different biological origin such as bacteriocins.

According to the first aspect of the invention, the synthetic phage-like particles preferably are based on and/or derived from one or more naturally occurring phages.

Preferably two or more tail fibers ca emanate from the some position on the baseplate.

The synthetic phage-like particles provided by the method of the invention can comprise different functional sites, wherein a functional site is preferably selected from head (capsid), tail, spike, sheath, tube, baseplate, and tail fiber components of a bacteriophage.

Tail fiber functional sites are in particular preferred.

As used herein a "different tail fiber type" or "different type of tail fiber" refers in particular to different tail fibers which are derived from different bacteriophages and/or have been engineered in a different way and/or have different functional sites.

The synthetic phage-like particles may comprise a given and/or preselected functional site, i.e. a type of functional site, several times, e.g. one to six times, such as a fiber tail. For example, if the functional site comprising homologous proteins is a tail fiber there are usually one to six, preferably six, corresponding functional sites in the synthetic phage-like particles.

In some embodiments, the synthetic phage-like particles can comprise a functional site that is a head (capsid) or at least capsid fragments.

The synthetic phage-like particles may comprise a given type of functional site at least two times, e.g. like a fiber tail and is preferably selected from the group comprising tail fiber proteins or tail fiber loops, in particular receptor binding proteins. A tail fiber may be composed of homologous proteins including receptor binding proteins. For example, the long tail fiber of E. coli phage T4 is composed of four different proteins, wherein the distal subunit of the fiber, gp37, mediates receptor binding with its carboxy-terminal region.

In a preferred embodiment at least one tail fiber or at least one receptor binding protein of the synthetic phage-like particle is derived from a bacteriophage that differs from the bacteriophage providing one or more other tail fibers and/or functional sites.

The synthetic phage-like particle may comprise the same functional site for example, tail fiber proteins, tail fiber loops, binding proteins derived from at least two different bacteriophages, preferably synthetic bacteriophages. Tail fiber proteins are in particular preferred.

The synthetic phage-like particle preferably resembles a functional bacteriophage tail-like structure; in other words, the multi-protein structure is preferably a headless synthetic bacteriophage or a headless engineered bacteriophage. As understood herein such a synthetic phage-like particle can induce cell death without replication of the bacteriophage, for example by damaging the membrane potential.

The tail fibers of bacteriophages and correspondingly the tail fibers of the phage-like particles of the present invention are typically not individually attached to the baseplate. Instead, they usually self-assemble into bundles comprising up to four, but preferably three tail fibers. Such a tail fiber bundle may resemble an arm-like structure that emanates from or is attached to a shoulder-like structure on the baseplate.

The inventors have surprisingly found that the target range of synthetic phages and synthetic phage-like particles can be extended by providing tail fiber bundles composed of tail fibers specific for different target cells.

According to one embodiment of the invention all tail fibers of a tail fiber bundle have the same specificity.

According to another embodiment, at least one tail fiber of a tail fiber bundle binds specifically to another target.

The synthetic phages or synthetic phage-like particles comprise up to ten tail fiber bundles attached to specific shoulder-like structures on the base plate. It is preferred that 4-6 tail fiber bundles are attached to the shoulder-like structure on the base plate.

Despite differences in the shape of functional tail-like structures, the synthetic phage-like particle of the invention is generally based on self-assembly or guided assembly of bacteriophage proteins or other chaperons.

The synthetic phage-like particle, preferably a synthetic headless bacteriophage, is designed to be self-assembled. In this context, 'self-assembled' refers to a multi-peptide complex that spontaneously forms a structural organization or pattern through the specific interactions of its constituent proteins. This structural organization might resemble a synthetic headless bacteriophage that contains all the components of a wild-type bacteriophage except for the capsid proteins. Alternatively, it could be a synthetic headless bacteriophage that lacks not only capsid proteins but also other phage proteins such as spikes, sheaths, tubes, baseplates, and/or tail fibers. The proteins in the synthetic phage-like particle are designed to self-assemble via specific interactions. Such interactions may be non-covalent or covalent, wherein non-covalent interactions are preferred. The self-assembly process may be facilitated by chaperones or similar assisting molecules.

The synthetic phage-like particle, which is preferably a synthetic headless bacteriophage, is preferably capable of binding on the surface of a cell, in particular a bacterial cell. At least one of the comprised tail fiber proteins may be specific for an epitope "E1" while at least one comprised tail fiber protein may be specific for an epitope "E2", wherein being specific for different epitopes preferably indicates specificity for different target cells. The synthetic phage-like particle may therefore be called multi-functional.

"Host" or "host cell" as used herein describes the whole breadth of organisms comprising single species as well as strains which can be affected by the synthetic phage-like particle of the present invention.

"Host" or "host cell" expresses a relationship in natural systems encompassing replication and expression of a phage within the specifically recognized host cell, as well as the potential release of a new phage generation from the host cell.

It is apparent to those skilled in the art that such relationship can only be incompletely transferred to synthetic phages with specificity for multiple host cells. For example, if the host cell recognition is extended by use of synthetic tail fibers to an additional cell or switched entirely to a different target cell, this does not automatically entail expression, assembly, and/or release of a new phage generation from the then-recognized cells, as these capabilities are linked in a much more complex manner with the cell's expression machinery. It may be, for example, even desirable to avoid the release of a new phage generation, for example to avoid a toxin overflow.

When using synthetic bacteriophages, which lack the genetic material for the replication of a new phage generation, these abilities may be completely lost, rendering the term "host cells" somewhat unjustified in this context. It should also be noted that the term "host cell" is undoubtedly misleading in the context of bacteriocins, which generally lack the ability to replicate in the recognized target cells.

Therefore, in connection with the synthetic phage particles of the present invention, the term "target cell" is preferably used instead of the term "host cell".

In a preferred embodiment, all proteins required for constructing the synthetic phage-like particle with specificity for two target cells are expressed in a host cell and self-assemble, or assemble guided or assisted by co-factors, into a synthetic phage-like particle that can specifically bind to cell surface markers "E1" and/or "E2".

In further preferred embodiments, the synthetic phage-like particle exhibits specificity for more than 2 different epitopes, particularly for 3, 4, 5, 6, 7, 8, 9, or 10 different epitopes ("E3"-"E10"), wherein the synthetic phage-like particle comprises a multi tail fiber system comprising a plurality of tail fibers preferably 1, 2, 3, 4, 5, 6, 7, 8, 9, or 10 different tail fibers ("TF1" - "TF10") characterized by specifically binding cognate epitopes for example epitopes "E1"-"E10" and wherein individual tail fibers are encoded by individual tail-fiber genes.

Since the synthetic phage-like particles are based on naturally occurring phages, phage-like particles with six or four tail fibers (corresponding to T4 or Lambda phages) are preferred. When filamentous phages, often described to comprise tail-fiber-like structures that differ in number and arrangement from T4 or Lambda, are used as the basis, synthetic phage-like particles with specificity for 2 to 10 epitopes are also preferred.

As herein understood tail fiber proteins, in particular receptor binding proteins, may be specific for any suitable target. For example, tail fiber proteins may be specific for gram positive and/or gram-negative bacteria, or even mycobacteria. Thus, a skilled person, utilizing synthetic tail fiber proteins, can enhance the target range to include cells of higher organisms such as plant or animal cells, particularly mammalian cells, for instance, human cells.

The tail fibers with distinct target specificity may be derived from different bacteriophages, in particular different wild-type bacteriophages. However, they can also be derived from the same bacteriophage, wherein at least one peptide has been engineered. The synthetic phage-like particle may be derived from the same wild-type but engineered differently.

Different bacteriophages where the tail fibers are derived from, in particular wild-type bacteriophages, may be selected from the group comprising Caudovirales (familiy of Myoviridae, Autographiviridae, Podoviridae, Ackermannviridae, and Siphoviridae), Leviviridae, Tectiviridae, Corticoviridae, Plasmaviridae, Sphaerolipoviridae, Inoviridae, Microviridae, Picobirnaviridae and/or Cystoviridae.

According to a preferred embodiment, the synthetic phage-like particle is encoded by a bacteriophage derived nucleic acid, in particular a bacteriophage genome.

Such bacteriophage derived nucleic acid may be any genetic material of bacteriophages, in particular bacteriophage genome, which can be either DNA or RNA and/or variants thereof, which can either be double-stranded or single-stranded. Such genetic material or nuclear material of bacteriophages, in particular of a bacteriophage genome, may also be engineered, or the derived nucleic acid is an unmodified bacteriophage genome, in particular wild-type genome.

Engineering, e.g. peptide engineering, may expand the abilities of the underlying protein, in particular tail fiber protein, by approaches known to the person skilled in the art and includes in particular site-specific mutations such as insertions, deletions and/or point mutations of the corresponding protein encoding nucleic acids, exchange of complete parts, incorporation of non-natural amino acids as well as the addition of further functional groups such as labeling groups, active agents etc. As understood by the person skilled in the art such engineering can be performed on the nucleic acid level as well as on the protein level.

Due to the degeneracy of the genetic code, any nucleic acid sequence can be varied or changed without substantially affecting the sequence of the protein encoded thereby, to provide a functional variant thereof. Suitable nucleotide variants are those having a sequence altered by the substitution of different codons that encode the same amino acid within the sequence, thus producing a silent change. Other suitable variants are those having homologous nucleotide sequences but comprising all, or portions of, sequence, which are altered by the substitution of different codons that encode an amino acid with a side chain of similar biophysical properties to the amino acid it substitutes, to produce a conservative change. Such a conservative change may be, for example, done for improved expression of the protein in a given expression system.

According to a preferred embodiment, the synthetic phage-like particle comprises receptor binding proteins comprising non-naturally generated mutations, e.g. resulting from deletions, insertions and/or substitutions within the corresponding encoding nucleic acid as described above.

According to a preferred embodiment the synthetic phage-like particle, in particular headless phage, may be labelled. Such a label may be, for example, a marker, or an active agent. A "marker" as understood herein may be a tag such as biotin, a his-tag or any other tag recognizable by a binding partner such as an antibody, useable, for example, to isolate the synthetic phage-like particle and/or tags masking the synthetic phage-like particle from the immune system of an object to be treated, such as peptides of human cells to mask the synthetic phage-like particle from the human immune system. Markers suitable for use in affinity purification processes include glutathione-5-transferase (GST), protein A, ScFv and lectins. A marker may be also a dye or a fluorescent label, e.g. for use in detection assays. Other modifications of the synthetic phage-like particle may be made, e.g. for modulating antigenicity, including use of a PEG (polyethyleneglycol) conjugate or a polysialic acid conjugate. An "active agent" as understood herein enhances the lethality of the synthetic phage-like particle to the target cell. The label may be incorporated at the DNA level, may be attached chemically at proteins of synthetic phage-like particle or may even self-assemble into the inventive synthetic phage-like particle, preferably within the expression in a cell free system.

In a preferred embodiment, all proteins required for the construction of the synthetic phage-like particle are encoded on a single nucleic acid. This nucleic acid largely resembles a phage genome but is manipulated (i.e. synthetic) as it comprises at least two genes that code for individual tail fiber proteins. Genes that are present but not required for the production of a specific synthetic phage-like particle, such as genes coding for capsid, are preferably deleted or their expression is prevented by methods known to the person skilled in the art for example by targeted silencing of the coding regions using siRNA, miRNA, or by altering the coding sequence to introduce frame shifts or stop codons.

In a preferred embodiment, at least one tail fiber is encoded by an individual nucleic acid molecule, with preferably more tail fibers for example two, three, four, five, six, seven, eight, nine or ten tail fibers being encoded on separate nucleic acids.

According to one embodiment, all proteins required for the construction of the synthetic phage-like particle are encoded on individual nucleic acids.

The term "protein encoding nucleic acid" is well understood by the persons skilled in the art and refers to a segment of nucleic acid which can either be DNA or RNA that contains the genetic information necessary for the synthesis of a protein. In the context of molecular biology and genetics. In essence, the protein-encoding nucleic acid carries the genetic code or sequence that is translated into a corresponding protein. In eukaryotic organisms, such as humans, this process involves transcription of DNA into mRNA (messenger RNA) and then translation of mRNA into a specific sequence of amino acids, forming a protein. In a basic form, a nucleic acid encoding a protein comprises a promotor, a start codon, the open reading frame of the coding region, and a stop codon.

However, the person skilled in the art is well aware that a nucleic acid encoding an amino acid can be provided by various means, comprising circular or linear forms of either single or double stranded DNA or RNA, including PCR products, plasmids or vectors, e.g. based on modified chromosomes or by means of providing whole naturally or artificial chromosomes or genomes. For the production of inventive synthetic phage-like particle, a nucleic acid can for instance be provided which codes for a fusion protein on the basis of already fused "open reading frames".

However, the proteins involved in the formation of a synthetic phage-like particle can also be expressed in parallel or sequentially, both mono- and/or poly-cistronic, and can assemble into synthetic phage-like particle independently or by means of additionally provided co-factors such as chaperones.

Many suitable methods for the provision and modification of coding nucleic acids are available in the art. One of the more recent developments comprises the CRISPR/Cas9 technology. A revolutionary gene-editing technology that allows for precise modification of DNA. The CRISPR system utilizes RNA molecules to target specific DNA sequences, and the enzyme Cas9 cuts the DNA at those locations. This cut triggers the cell's repair mechanisms, enabling the introduction of genetic changes. CRISPR/Cas9 is employed in genome editing, genetic research, and the development of novel therapies.

By way of example, for the provision of encoding nucleic acids, reference is made to one of many widely recognized publications "Molecular Cloning: A Laboratory Manual" by Michael J. Sambrook and David W Russell.

Preferably, the DNA and/or RNA molecules for use in the production of synthetic phage-like particle essentially based on complete genomes of different bacteriophages or protein encoding regions thereof, preferably phage tail fiber proteins.

In a preferred embodiment the synthetic phage-like particle comprises a bacteriophage derived nucleic acid, in particular bacteriophage genome encoding at least one functional site for example tail-fiber, baseplate or capsid. If the synthetic phage-like particle comprises no capsid proteins a nucleic acid encoding tail-fiber or baseplate proteins is preferred.

It is further envisaged that the nucleic acid of at least two different wild-type bacteriophages, a first wild-type bacteriophage and at least one mutated or engineered bacteriophage derived from said first wild-type bacteriophage or at least two mutated or engineered bacteriophages derived from the same wild-type bacteriophage is provides for use in the production of synthetic phage-like particle of the invention. Varying the concentration of the thereby added coding genetic information, it is possible to adjust e.g. the tail fiber composition of the resulting synthetic phage-like particle for further use.

When providing protein-encoding nucleic acids of at least two different bacteriophages the encoding nucleic acid may be modified, in particular modified by non-naturally mutations such as deletion, insertion and/ or substitution as described herein above.

The term "epitope" refers to a site on a target such as a surface protein on a cell or a lipopolysaccharide or peptidoglycan of a bacterial cell wall to which a binding protein in particular a tail-fiber specifically binds.

In the context of the invention, each tail-fiber is specific for a single corresponding epitope. However, there are numerous distinguishable targets on the surface of cells or bacteria to which individual tail fiber or binding proteins can bind.

According to the invention, a synthetic phage-like particle having a specificity for two or more than two different epitopes can therefore, in certain embodiments, bind to two or even more than two epitopes on the surface of a cell, which enables modulation of the specificity of the synthetic phage for its target cell.

In a preferred embodiment, a synthetic phage-like particle binds with each specific binding protein, which is preferably a tail-fiber, to an epitope of a target on the surface of different cells.

The respective synthetic phage-like particle is therefore multi-specific with respect to its target- or target-cells, which are preferably bacterial target cells.

Bacteriocins are a class of protein or peptide antibiotics produced by bacteria to inhibit or kill closely related or even different bacterial species, a biological process often referred to as microbial interference. These substances therefore play a significant role in microbial competition and can be used as natural preservatives or in medical therapy to combat antibiotic-resistant bacteria.

Although bacteriocins and bacteriophages are different entities, there are some interesting parallels. Both can target specific bacterial strains. Unlike bacteriophages, which are viruses that infect and lyse bacterial cells, bacteriocins are proteins or peptides that damage their target cells through various mechanisms without infecting them, e.g. by dissipating the cell's proton force, interfering with DNA or RNA replication or protein expression.

While Class I and II bacteriocins with a broad spectrum of activity resemble classical antibiotics, Class III and IV bacteriocins act very specifically due to structures for surface recognition, similar to phages. Some Class III bacteriocins can even be traced back to prophages, indicating a genetic link between some bacteriocins and bacteriophages even though most of the microbial interference proteins and their coding nucleic acids have evolved independently of phages.

Class III comprises for example bacteriocins produced by E. coli which target specific receptors on the surface of other E. coli strains. These "colicins" can utilize different mechanisms to kill their target cells, including pore formation in the cell membrane or inhibition of DNA, RNA and protein synthesis.

Class IV refers to complex bacteriocins, consisting of multiple protein components and may contain both protein and lipid or carbohydrate parts of which tailocines represent the most studied examples.

Tailocines are proteinaceous particles morphologically similar to phage tails with *Pseudomonas aeruginosa* tailocines coined F-type (flexible) and R-type (rigid) pyocines being models for phage-like bacteriocines that target specific bacterial receptors and initialize killing by inserting their tube into the targeted cell. However, morphologically similar bacteriocins have been identified in several other γ-proteobacterial genera, such as the phytopathogen *Pectobacterium carotovorum* causing soft rot, xenorhabdicin of the entomopathogenic nematode symbiont *Xenorhabdus nematophila*, and maltocin of the opportunistic pathogen *Stenotrophomonas maltophilia* (Ghequire & DeMont; 2015; Trends in Microbiol).

Further prominent members of Class iV are 'diffocins'. They are produced by certain strains of Clostridium difficile, a species known for causing severe gastrointestinal infections.

These bacteriocins are high molecular weight complexes and have a contractile tail structure similar to the R-type pyocins of Pseudomonas aeruginosa. They target specific strains of C. difficile and initialize killing of the target cells by puncturing the cell envelope, similar to the action of R-type pyocins, thereby providing a competitive advantage to the producing strain.

Not only do tailocines share striking morphological similarity, including a similar mass of 10⁵-10⁷ Da with phage tails, but it has further been shown (S.R. Williams et al. Appl. Environ. Microbiol 2008) that the lipopolysaccharide-mediated targeting spectra of R-pyocin, can be altered by the construction of chimeric tail fibers.

Without being bound by theory, this demonstrates for class 3 and 4 bacteriocins in general, and in particular for tailocins such as F- or R-type pyocins, that synthetic phage-like particles of the invention can be based on bacteriocins.

A synthetic phage-like particle of the invention is therefore preferably a synthetic headless phage or a synthetic bacteriocin, selected from the group comprising a synthetic colicin, tailocin, diffocin, monocin or pyocin, preferably a synthetic tailocin, diffocin or pyocin.

Since the synthetic phage-like particles of the invention are not exclusively based on phages rather, some of them structurally build on bacteriocins in specific embodiments. Therefore, the methods by which the synthetic phage-like particles bind to target cells and initiate killing are diverse. A common feature of all these mechanisms is that the initial binding of the synthetic phage-like particle to the target cell causes structural changes that often occur in the tail-like structure of the synthetic phage and/or in the cell envelope of the target cell, which preferably is the cell wall and membrane of a bacterium or the cell membrane of a higher cell, thereby perforating said cell envelope and allowing e.g. the transfer or translocation of compounds associated to the synthetic phage like structure to be translocated into the target cell.

Preferably, the binding of the synthetic phage-like particle to its corresponding target cell comprises a perforation of the cell envelope of the target cell, which may initialize death of the target cell.

In specific embodiments, this translocation is not associated with permanent perforation of the cell but is rather accomplished through a transport mechanism of the target cell, as exemplified by *Colicin N*, which is introduced into the target cell via receptor-mediated translocation. Especially in higher cells, numerous active transport mechanisms are known that can be specifically targeted to actively transport substances into the cell.

In a specific embodiment, the binding of the synthetic phage-like particle to its cognate target is accompanied by the translocation of a compound across the cell envelope. This compound is preferably a lytic compound, a compound inhibiting the growth of the target cell, or a compound associated with the synthetic phage-like particle for targeted delivery to the target cell.

In a specific embodiment, the translocation of a component across the cell envelope involves an active transport process where a transport system of the target cell is involved or entirely carried out by a transport system of the target cell.

The synthetic phage-like particle of the invention may be a targeted delivery system designed for the precise administration of compounds, preferably therapeutic agents, into target cells, wherein the system comprises a carrier component, structurally analogous to phage-like particles, which exhibits specificity for binding to specific epitopes on the target cell.

The synthetic phage-like particle of the invention may be a targeted delivery system designed for the precise administration of compounds, preferably therapeutic agents, into target cells, wherein the system interacts with a transport system of the target cell.

The term 'cell envelope' generally refers to the often multi-layered structure that surrounds and protects a cell. More specifically, the term is commonly applied to bacterial cells, where it includes the combination of the cell wall and plasma membrane, which act together to provide the cell with structure and protect it from external influences. In this context, it applies regardless of the specific composition of this outer barrier, for example, to Gram-positive, Gram-negative, mycobacteria, and also archaea.

In the context of the present invention, which discloses synthetic multi-specific phage-like particles that are not necessarily based on phages, the term 'cell envelope' is understood to encompass all external structures that surround target cells recognized by the synthetic phage-like particles of the invention.

In particular, within the context of this invention, the term 'cell envelope' refers to the cell wall and plasma membrane of plant cells, which together form a robust barrier that not only provides support and protection but is also involved in regulating substance exchange, as well as to cells of higher organisms that lack a cell wall and are instead bounded by a dynamic phospholipid bilayer.

### Composition of inventive phage-like particles

The expression of a mixture of tail fiber-encoding nucleic acids along with proteins necessary for the construction of a functional tail and baseplates may lead to the formation of synthetic phage-like particles with varied tail-fiber compositions.

These include particles with individual tail fiber proteins here for illustrative purposes referred to as 'TF1, TF2, TF3' as well as those with combinations of these proteins (TF1/TF2, TF1/TF3, TF2/TF3, or TF1/TF2/TF3). Notably, the ratio of tail fiber proteins in the mixture of particles, especially when expressed in a cell free system, may mirror the ratio of therefore provided coding nucleic acids.

The inventors surprisingly discovered that modulating the amount of encoding nucleic acids allows altering the ratio of tail fibers in the mixture produced by the cell-free expression, thereby also enabling the modulation of the specificity of the particle mixture. The specificity of a mixture comprising a plurality of different phage particles can thus be adjusted quickly and in a targeted manner.

A second aspect of the invention therefore relates to a composition comprising a plurality of synthetic phage-like particles having the specificity for at least two different target cells, wherein the synthetic phage-like particles differ with respect to the type and/or number of different tail fibers in different tail fiber systems.

Such composition may be characterized by a predetermined and/or preselected ratio of tail fiber types.

The ratio of different tail fiber types may be adjusted and/or preselected by providing corresponding tail-fiber encoding nucleic acids in corresponding predetermined amounts and/or ratios for tail fiber expression, i.e. in particular in the cell free expression system which is in the context of this invention preferably used.

According to the invention, once surface markers, in particular epitopes on pathogenic target cells are identified, a therapeutically relevant, multi-cell-specific synthetic phage composition can be produced by co-expression of suitable - preferably selected based on the predetermined surface markers - nucleic acids, optionally in a cell-free system. This process entails the co-expression of (i) nucleic acid of a regulatory safe synthetic phage preferably encoding baseplate and/or tail-fiber proteins and (ii) a plurality of nucleic acids encoding relevant tail fibers (TFs) optionally in a cell-free system, followed by subsequent assembly of respective synthetic phage particles and isolation of the composition.

The composition comprising a plurality of synthetic phage-like particles preferably comprises a predetermined ratio of individual tail fibers wherein the number of individual tail fibers in the composition can be modulated based on the varying amounts of nucleic acids encoding individual tail fibers or, depending on the expression system used, by selecting suitably strong promoters to control the expression of individual tail fibers. In addition to modulating promoter strength, the person skilled in the art is familiar with numerous other methods for regulating expression, which include the use of repressors and silencers, as well as employing siRNA or miRNA, for example.

In a preferred embodiment, the composition of synthetic phage-like particles comprises individual synthetic phage-like particles, each equipped with a tail-fiber system which optionally comprises a plurality of individual tail fibers. It is preferred that both, the total number of tail fibers in the composition and the number of tail fibers per particle are modulated.

It is preferred that the amount and ratio of individual tail fibers in the composition of synthetic phage-like particles are adjusted by the provision of predetermined amounts of respective tail fiber-encoding nucleic acids.

In addition to modulating the number of nucleic acids or the expression of tail-fiber coding nucleic acids, using a cell-free system in particular offers the advantage of adjusting the retention time of a nucleic acid or the specific processing step at which an individual nucleic acid is provided.

Accordingly, it is preferred that the ratio and/or number of individual tail-fibers in a composition of synthetic phage-like particles is modulated by providing tail-fiber encoding nucleic acids in predetermined amounts and/or at a predetermined stage during the cell free production of the Composition.

An advantage of a synthetic phage composition is certainly the simple and rapid preparation of the mixture with a predetermined tail-fiber composition. Preferably, this mixture has a broader or improved target cell specificity compared to individual phage-like particles. In the context of the invention, an improvement in specificity does not always imply extending the target range; for example, improving may also imply higher specificity for a target cell by binding multiple times to different epitopes on the same target cell.

Accordingly, it is preferred that the composition of the synthetic phage-like particles has an enhanced and/or improved target specificity.

A further preferred embodiment therefore relates to a synthetic phage composition produced by the above-described method.

### Production in a cell free system

In addition to the synthesis of synthetic phage-like particles in host cell systems, the synthesis of synthetic phage-like particles in cell-free systems, especially in a method for continuous expression, has proven to be particularly well suited.

These methods are mostly based on methods developed for the cell-free production of viruses, bacteriophages or multi-protein complexes and are described in detail in (e.g. WO 2023/170076). The contents of which are hereby incorporated in their entirety.

The cell-free production offers several advantages as it for instance avoids the need for host bacteria, allowing to produce phage based particles at lab scale without the need to comply with increased biosafety-precautions. At the same time the number of toxic by-products that are produced is significantly reduced, since lysed pathogenic host bacteria are not present.

Furthermore, in the cell-free system, the absence of cell walls significantly facilitates the provision of coding nucleic acids and co-factors for expression or assembly at any desired point during the expression process. Rapid prototyping of synthetic phage-like particles therefore often depends solely on the availability of suitable coding nucleotide sequences and can usually avoid time and labor-intensive cloning steps.

It is therefore preferred that the synthetic phage-like particles of the present invention are expressed in a cell-free system.

The terms "cell-free expression system" or "cell-free protein synthesis system" (CFPS system) are well understood by a person skilled in the art and refers in general to an in-vitro method that allows the synthesis of proteins outside of living cells.

CFPS systems are not only suitable for the production of individual proteins or protein moieties, but also for the parallel production of multiple distinct proteins that are either independent of each other or assemble into higher multiprotein units such as synthetic phage-like particles, in particular headless phages of the invention. In the context of the present invention, the CFPS system is a preferably host independent system. The term "cell free" is understood by the person skilled in the art and refers to "substantially free of".

A CFPS system as understood herein comprises a complete transcription and translation machinery for transcription and expression of nucleic acids encoding synthetic phage-like particles, in particular a headless phage of the invention.

A preferred example for a CFPS system is a cell lysate. Using such a cell lysate it is possible to synthesize several proteins or metabolites at the same time.

"Cell lysate" according to the present invention refers to a fluid comprising the components of cells from which it is derived after lysis. Lysis methods are known to the person skilled in the art and break down the membrane of a cell, for example, by viral, enzymatic, or osmotic mechanisms that compromise its integrity. Such cell lysate is essentially void of intact cells, i.e. cell-free. The terms "cell lysate" and "cell extract" can be used herein interchangeably. After purification this lysate is essentially free, preferably free of host DNA and makes an expression of the desired protein possible by the external addition of DNA, in particular a amplified bacteriophage genome as herein described. Thus, the used cell lysate is preferably free of nucleic acids, in particular DNA, and/or membranes from the cells of which it is derived. In a preferred embodiment, using standard techniques no host DNA can be detected in the purified lysate.

Cell lysates used according to the present invention may be derived from microorganisms in particular bacteria such as non-pathogenic or pathogenic bacteria (for example E. coli), imperfect or perfect fungi such as yeast, or from eukaryotic cells such as insect, mammalian and/or plant cells (in particular wheat or rice), or may be even artificially. produced. "Microorganism" refers to a bacterium or an archaeon. Preferably, the microorganism is a bacterium.

A number of cell-free expression systems are known to a person skilled in the art und may be applied according to the present invention. For example, the "PURE" system consists of several isolated proteins (Shimizu et al.) while untreated cell lysates (crude extracts) such as of E. coli include almost all intracellular proteins, even those that are not necessary for expression.

The use of E. coli lysate, in particular crude E. coli lysate, is a further preferred embodiment. A preferred example of crude E. coli lysate is E. coli S30 cell extract produced by a method based on the protocol described in E. Falgenhauer et al. (Falgenhauer, S. von Schönberg, C. Meng, A. Möckl, K. Vogele, Q. Emslander, C. Ludwig, F. C. Simmel, ChemBioChem 2021, 22, 2805).

External factors such as energy carriers (e.g. 3-phosphoglyceric acid), co-factors, natural and/or non-natural amino acids, polymerases, transcription regulatory factors, chaperons, DNA stabilization agents (e.g. GamS and/or Chi6 DNA) may be added to enhance or otherwise improve the reaction. Such factors may be added as proteins and/or as nucleic acid encoding a respective protein.

According to a preferred embodiment, polyethylene glycol (PEG, in particular PEG 8000) and/or Ficoll (e.g. Ficoll-400) may be added to the cell extract to increase molecular crowding. PEG binds water, creating a more concentrated environment. This may be advantageous because there is up to 25 to 30 times less protein concentration in the cell extract than in the cytosol of a bacterial cell.

If the cell-free lysate to be used is derived from a cell or microorganism which is different to the natural host on which the synthetic phage-like particles is based, at least one host specific expression factor and/or a nucleic acid encoding the at least one host specific expression factor, preferably a transcription factor, may be added, if necessary. Host specific expression factors in particular Bacteriophage specific host factors" or "bacteriophage-host specific expression factors" with regard to a given bacteriophage are well known to the person skilled in the art and/or can be easily determined (cf e.g. Role of host factors in bacteriophage φ29 DNA replication, Adv Virus Res 2012; 82:351-83 or US2022025957A1).

Accordingly, when expressing a synthetic phage-like particles of the invention, at least one nucleotide sequence encoding the at least one host specific expression factor or any other protein preferably selected from the group comprising co-factors, chaperons, polymerases, transcription regulatory factors is cloned into the cell strain used to provide the cell lysate. The nucleotide sequence is expressed within the cell before lysis. Thus, after lysis the cell lysate already contains all nucleic acids and/or proteins necessary for the amplification of the virus or bacteriophage on which the synthetic phage-like particles of the invention are based, in particular at least one host specific expression factor or any other protein selected from the group comprising co-factors, chaperons, polymerases, transcription regulatory factors and/or any mixture thereof.

Further preferred modifications of the CFPS system include the addition of one or more modified proteins or the addition of a nucleic acid encoding these proteins. These proteins may have a natural amino acid sequence or may be artificially modified for optimization and may be incorporated into the multiprotein complex, which is preferably a bacteriophage, or may modify it, e.g. by an enzymatic modification such as glycosylation.

Although cell-free production offers many advantages over production within a cell, cell-free protein synthesis (CFPS) using a single-compartment system appears to be limited, both in terms of protein yield and the efficient modification and purification of products.

A method overcoming these limitations is the cell-free continuous expression, whereby translation occurs in the inner compartment of a reaction chamber comprising the inner fluid comprising the cell-lysate or cell-extract and the template nucleic acid, and wherein assembly, modification and/or purification of the synthetic phage-like particle optionally takes place in the outer compartment comprising the outer fluid.

In the context of the continuous CFPS the inner compartment preferably comprises the CFPS system or parts of the CFPS system. Missing or consumed or depleted substances can be added at any time, usually depending on the state of the reaction, preferably by means of a supply stream so that the protein synthesis is maintained over a prolonged period of time in order, for instance to increase the yield of the end product, such as the synthetic phage-like particle or composition thereof.

To enable maintaining the synthesis inside the compartment throughout the incubation period, protein expression needs replenishment of consumed substances such as nucleotides or energy carriers. The skilled person can deduce which compounds are required at a given time by monitoring the reaction or from known production kinetics. It is often preferred that the replenishment is realized by means of a supply stream running through the inner compartment, or by a supply stream running past the outer boundary surface of the compartment.

Which substances can be exchanged from one compartment to another using the continuous expression method depends on the specific material properties of the boundary surface. The person skilled in the art will tailor the boundary material to the method. This customization ensures that all necessary substances can be exchanged across the boundary during production. The goal is to produce the desired protein. In some cases, this process may also involve the exchange of the protein to be produced itself.

Substances preferably exchanged or replenished over the membrane separating the compartments preferably comprise the provided protein itself but also external factors such as energy carriers, co-factors (likewise PEG e.g. to increase molecular crowding), amino acids (natural occurring and/or synthetic amino acids comprising nuclease resistant and labeled amino acids), polymerases, ligases, transcription regulatory factors, chaperons, DNA stabilization agents (e.g. GamS and/or Chi6 DNA). Such factors may be added as proteins and/or as nucleic acid encoding a respective protein.

Preferred are also substances and components enabling post-translational protein modification such as glycosylation, acylation, alkylation, carboxylation, hydroxylation, methylation, myristoylation, palmitoylation, isoprenylation, lipoylation or substances for the specific labelling of proteins such as chemical dyes or protein tags (e.g. affinity or snap tags) to be exchanged via the membrane as required.

Proteins produced in the reaction chamber are preferably exchanged across the membrane separating the compartments and are preferably isolated with high purity from the outer fluid or interact with substances provided therein. Preferably, specific post-translational modifications and/or labelling are added to the provided protein which is preferably a synthetic phage-like particle in the outer fluid.

The two compartments are preferably separated by a membrane, the cut-off value of which can be chosen to allow an efficient separation of the substances required for the expression of the synthetic phage-like particles.

In a preferred embodiment, the expression, translation, and optional assembly of the phage-like particles can take place inside the compartment. Expressed proteins preferably translocate to the outer compartment, where they optionally assemble and undergo optional post-translational modifications.

In a preferred embodiment, the expression, translation, and optional assembly of the synthetic phage-like particles takes place inside the inner compartment, where they are fitted with an affinity tag. Subsequently, the expressed proteins preferably translocate to the outer compartment, where they can optionally be supplemented with a component presented in the outer chamber by means of the affinity tag.

It is preferred that, this supplementation for example, comprises attaching a tail fiber to the synthetic phage like particle or associating the synthetic phage-like particle with a component provided for targeted delivery to a specific cell.

In the context of continuous expression, it is for example conceivable to carry out the expression of proteins for the production of a synthetic phage-like particle in the inner compartment and to provide additional compounds in the outer compartment that associate with the translocated and optionally assembled synthetic phage-like particle in the outer compartment. These compounds can then be specifically delivered to the target cell by the synthetic phage-like particle.

In a preferred embodiment, in the continuous expression method, the components required for the assembly of the synthetic phage-like particles are supplied continuously or successively during the expression by means of a supply stream. Preferably, the components are provided in the outer fluid and translocate across the membrane into the inner compartment.

Utilizing the supply stream, it is preferably possible to produce high concentrations of synthetic phage like particles which can optionally be isolated from the inner compartment or from the outer compartment.

The production of a synthetic phage-like particle, regardless of the methodology used - whether in a cell-free system or utilizing cell-based production - is carried out at temperatures below 37°C, typically in the range of 20°C to 30°C. In other embodiments, however, lower temperatures such as 4°C are also possible.

The synthetic phage-like particles of the invention or composition thereof is provided preferably in an isolated form. Corresponding techniques for isolating or purifying are known to the person skilled in the art. The provided synthetic phage-like particles or compositions thereof can be purified and/or isolated from the inner fluid and/or the outer fluid, wherein the outer fluid is preferred.

The concentration of the synthetic phage-like particles of the invention or composition thereof may be at a level that the corresponding pharmaceutical composition and/or formulation can directly be applied in a therapy without a further concentration step. If needed, a further concentration step, in particular after purification, is of course also within the scope of the present invention.

A further aspect of the invention relates to a synthetic phage-like particle of the invention or composition thereof produced by a host-specificity modulating method of the invention.

The compositions comprising bacteriophages provided by the method of the invention may comprise carriers and/or vehicles as described below. According to preferred embodiments, the compositions are pharmaceutical compositions.

A further aspect of the invention relates to synthetic phage-like particles of the invention or compositions thereof as described herein for use in medicine, i.e. as a medicament, chemistry, biotechnology, agriculture and/or food industry. Such uses are not limited to bacteria related fields and comprise, for example, the use as vaccine, for example against infections as well as tumors, and the use as a delivery vehicle for any kind of drug, in particular anti-cancer drugs, and/or nucleic acids to a target cell, in particular a human target cell. A preferred embodiment relates to the use of synthetic phage-like particles of the invention or compositions thereof, provided as described herein for use as a medicament. According to certain embodiments the synthetic phage-like particles of the invention or compositions thereof, described herein may serve as vehicle or carrier for antigens to be delivered. For example, such antigen structures to be delivered may be attached to synthetic phage-like particle. The synthetic phage-like particles of the invention or compositions thereof, may serve as or provide vaccines, e.g. by delivery of antigens and/or carrying nucleic acids to be expressed.

The use in bacteria related fields is, however, preferred. Synthetic phage-like particles of the invention or compositions thereof, may in particular be used as bacterial population control alternatives to antibiotics. They are much more specific than antibiotics and are typically harmless not only to the target organism but also to other beneficial bacteria, such as the gut microbiota, as they are very selective in the strains of bacteria, they are effective against. Thereby the chances of opportunistic infections are significantly reduced. Advantages include further reduced side-effects and reduced risk of the bacteria developing resistance.

Accordingly, the synthetic phage-like particles of the invention or compositions thereof described herein may be part of a pharmaceutical composition. Such a composition may comprise (i) synthetic phage-like particles of the invention or compositions thereof (ii) a pharmaceutically acceptable carrier and/or vehicle. An inventive composition may also comprise (i) at least one synthetic phage-like particles of the invention or compositions thereof and (ii) a pharmaceutically acceptable carrier and/or vehicle and/or therapeutic agent. Of course, such compositions may comprise mixtures of synthetic phage-like particles. Preferred embodiments relate to such compositions for use as a medicament.

The pharmaceutical compositions may be used in combination with antibiotics for the purpose of treating bacterial infections and/or to treat antibiotic resistant bacteria. In particular, bacteriophages tend to be more successful than antibiotics where there is a biofilm covered by a polysaccharide layer, which antibiotics typically cannot penetrate. According to a preferred embodiment, the composition may be freeze dried.

"Pharmaceutically acceptable carrier" relates to pharmaceutically acceptable fillers or diluents used to formulate pharmaceutical compositions for animal or human administration. The pharmaceutical compositions may further comprise pharmaceutically acceptable auxiliary agents, and optionally other therapeutic agents. In particular for oral administration it is preferred to add an antacid, i.e. a substance which neutralizes stomach acidity, thereby increasing the number of phages surviving passage through the stomach.

"Vehicle" as used herein refers to any compound or combination known to the person skilled in the art known to be useful in formulating a composition, in particular a pharmaceutical composition. Such a vehicle may include one or more substances which may also act as flavoring agents, lubricants, solubilisers, suspending agents, dyes, fillers, glidants, compression aids, inert binders, sweeteners, preservatives, dyes, coatings, or tablet-disintegrating agents. The vehicle may also be an encapsulating material.

In tablets, the active agent (i.e. multi-peptide structure, in particular bacteriophage) may be mixed with a vehicle having the necessary compression properties in suitable proportions and compacted in the shape and size desired.

Suitable solid vehicles include, for example calcium phosphate, magnesium stearate, talc, sugars, lactose, dextrin, starch, gelatin, cellulose, polyvinylpyrrolidone, low melting waxes and ion exchange resins.

In another embodiment, the pharmaceutical vehicle may be a gel and the composition may be in the form of a cream or the like.

The dose and regimen of administration of a pharmaceutical composition will necessarily be dependent upon the therapeutic effect to be achieved (e.g. treatment of IBD) and may vary with the particular bacteriophage strains in the composition, the route of administration, and the age and condition of the individual subject to whom the pharmaceutical composition is to be administered.

If the pharmaceutical composition is to be administered with one or more antibiotics, it may be simultaneously, separately or sequentially administered.

Pharmaceutical compositions and routes of administration include those suitable for or via oral (including buccal, sublingual and intraorbital), rectal, nasal, topical (including transdermal), ocular, vaginal, bronchial, pulmonary or parenteral (including subcutaneous, intramuscular, intravenous, intradermal, intraperitoneal, intrapleural, intravesicular and intrathecal) administration or administration via an implant. In particular a freeze-dried composition may be administered orally, preferably in form of a pill.

Agriculture comprises plant agriculture as well as animal agriculture, wherein bacteriophages can replace or complement antibiotics and/or pesticides.

Bacterial pathogens are associated with a couple of plant diseases which can also be treated with synthetic bacteriophages provided herein. Non limiting examples for bacterial plant diseases which might be treated are leaf blight on onion, potato scab or soft rot of potato, bacterial wilt on tobacco, citrus bacterial spot or citrus canker of citrus, fire blight on apple, black rot of cabbage, soft rot of calla lilies, bacterial wilt of tomato and bacterial spot of peach.

The use of synthetic phage-like particles of the invention or compositions thereof is in a general manner exemplified by the use against Salmonella, pathogenic E. coli, Clostridium, and Campylobacter for the poultry industry and against Salmonella and pathogenic E. coli for the pig industry.

Synthetic phage-like particles of the invention or compositions thereof may also be used to safen food products, and to forestall spoilage bacteria. Since 2006, the United States Food and Drug Administration (FDA) and United States Department of Agriculture (USDA) have approved several bacteriophage products. For example, LMP-102 (Intralytix) was approved for treating ready-to-eat (RTE) poultry and meat products. In that same year, the FDA approved LISTEX using bacteriophages on cheese to kill Listeria monocytogenes bacteria.

The invention further includes a kit comprising a pharmaceutical composition of the invention and instructions for the use of the composition for a use as hereinbefore described, optionally together with packaging material.

Further aspects relate to the use of synthetic phage-like particles of the invention or compositions thereof, provided by methods of the invention for the prevention and or treatment of a bacterial infection as well as for avoiding bacterial growth.

### EXAMPLES

The basis of the headless phage is the portal-tail complex and core of the T7 phage, especially without a capsid (namely gb10 the capsid protein).

The cell-free expression system is used for in vitro expression and assembly of the headless phages by supplying a DNA template based on the phage genome excluding essential parts of the capsid, like the major capsid protein gp10 of the T7 phage. The native tail-fiber gene is also missing to supply it externally in a modular way.

In addition, plasmids encoding for a tail fiber proteins can is added.

This co-expression of several different tail-fiber leads to the assembly of a headless phage with more than one tail fiber moiety. These headless phages differ from the mono-tail-fiber headless phage in their ability to attach to different hosts, thereby expanding the host range. The headless phages are mixed with the corresponding bacteria and incubated for the attachment of the headless phage particles to the bacteria.

Subsequently the bacteria are plated and the CFU/ml of each sample is measured and compared with the bacteria incubated with a cell-free reaction without DNA encoding for the tail-fiber. Based on this the fold reduction of the bacteria is calculated. The target of the T7 phage tail-fiber is *E.coli* NCIB 11595, of the T3 Phage tail-fiber the target strain is *E.coli* ECOR16, of the Bas Phage tail-fiber it is *E.coli* CGSC 7636 and for the phiYe03-12 the target is *Yersinia enterocolitica* bacterium Y.en. 6471/76. As a mode of action, the headless phage particles form a hole in the membrane of the target cell. This leads to a deregulation of the osmotic pressure of the bacterium and within a high osmotic medium, as used here, the bacteria are inactivated.

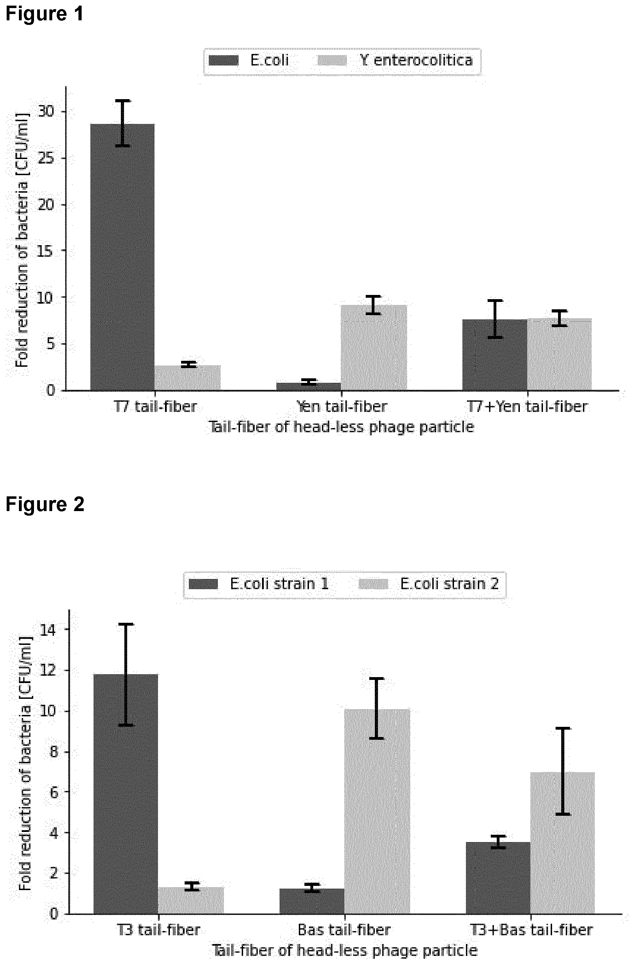

**Figure 1****:** Bar graph of the fold reduction of bacteria mixed with synthetic phage-tail like particles with different tail-fiber compared to a sample with a synthetic phage-tail like particle without a tail-fiber. (left) Cell-free expressed headless phage with a native T7 tail-fiber from an *E.coli* phage, (middle) cell-free expressed headless phage with the phiYe_p51 tail-fiber of the Yesinia phage phiYe03-12 and (right) cell-free expressed headless phage with both plasmids co-expressed. Co-expression of T7 and phiYe_p51 tail-fibers shows that the system can be used to successfully generate phages infecting 2 different species of bacteria (Figure 1).

**Figure 2****:** Bar graph of the fold reduction of bacteria mixed with synthetic phage-tail like particles with different tail-fiber compared to a sample with a synthetic phage-tail like particle without a tail-fiber. (left) Cell-free expressed headless phage with a native T3 tail-fiber from an *E.coli* phage, (middle) cell-free expressed headless phage with the Bas65_p50 tail fiber of the *E.coli* phage Bas and (right) cell-free expressed headless phage with both plasmids co-expressed. Co-expression of T3 and Bas65_p50 tai-fiber shows that the system can be used to successfully generate headless phages infecting 2 different strains of bacteria (Figure 2).

### Items

1. Synthetic phage-like particle, preferably a synthetic phage-tail like particle, having specificity for at least two different target cells.
2. The synthetic phage-like particle of item 1,
   wherein said synthetic phage-like particle comprises at least a functional tail comprising a baseplate and a multi tail fiber system,
   which multi tail fiber system comprises at least two different tail fibers having specificity for different target cells,
   wherein said tail fibers preferably emanate from said baseplate.
3. The synthetic phage-like particle of any of items 1 - 2, which is self-assembled.
4. The synthetic phage-like particle of any of items 1-3 having a mass of 10⁵- 10⁷ Da.
5. The synthetic phage-like particle of any of items 1-4,
   wherein the multi-tail fiber system comprises tail fiber bundles that comprise tail fibers, preferably up to 10, more preferably four, even more preferably three tail fibers, wherein the tail fibers in a tail fiber bundle may have specificity for the same or different target cells, i.e. wherein the tail fibers are different, e.g. engineered in a different way.
6. The synthetic phage-like particle according to any one of items 1 - 5,
   wherein the tail-fiber bundles comprise at least two different types of tail-fibers having specificity to different target cells.
7. The synthetic phage-like particle according to any one of items 1 to 6,
   wherein the base fiber bundles, preferably up to 10, more preferably 4 or 6 tail fiber bundles, emanate from of the baseplate.
8. The synthetic phage-like particle of any of items 1 - 7,
   wherein at least one tail fiber, preferably all tail fibers, is engineered.
9. The synthetic phage-like particle of any of claims 1 - 8,
   which is a synthetic headless phage or a synthetic bacteriocin preferably a synthetic tailocin, diffocin, monocin or pyocin.
10. The synthetic phage-like particle of any of items 1 - 9,
   wherein tail fibers are derived from bacteriophages selected from the group comprising Caudovirales (familiy of Myoviridae, Autographiviridae, Podoviridae, Ackermannviridae, and Siphoviridae), Leviviridae, Tectiviridae, Corticoviridae, Plasmaviridae, Sphaerolipoviridae, Inoviridae, Microviridae, Picobirnaviridae and/or Cystoviridae, or from bacteriocin producing bacteria selected from the group comprising Pseudomonas aeruginosa, Klebsiella and/or Clostridium difficile.
11. The synthetic phage-like particle of any of items 1-10,
   wherein the target cells are selected from the group comprising bacterial cells, such as E. coli, Pseudomonas and/or Yersinia, eucaryotic cells, plant and/or mammalian cells, in particular antibiotic resistant bacterial cells selected from the group comprising methicillin-resistant *Staphylococcus aureus* (MRSA), Vancomycin-resistant *Enterococcus* (VRE), multidrug-resistant *Mycobacterium tuberculosis* (MDR-TB), carbapenem-resistant *Enterobacteriaceae* (CRE), multidrug-resistant *Acinetobacter baumannii*, (multi-)drug resistant *Pseudomonas aeruginosa* and/or *Neisseria gonorrhoeae.*
12. The synthetic phage-like particle of any of items 1 - 11,
   which is provided by a cell-free system, preferably by a continuous cell-free expression system.
13. The synthetic phage-like particle of any of items 1 - 12,
   wherein the composition and/or ratio of different tail fibers of the multi-fiber system correlates with the composition and/or ratio of nucleic acids encoding different tail fibers present during tail fiber expression.
14. Composition comprising a plurality of synthetic phage-like particles of any one of items 1 - 13.
15. The composition according to item 14,
   wherein the composition comprises different synthetic phage-like particles with different tail fiber systems.
16. The composition according to items 14 or 15,
   wherein the synthetic phage-like particles differ with respect to the type and/or number of different tail fibers in different tail fiber systems.
17. The composition of any one of items 14 - 16,
   wherein the ratio of tail fiber types is adjusted and/or preselected by providing corresponding tail-fiber encoding nucleic acids in predetermined amounts and/or ratios for tail fiber expression.
18. System adapted for the production of a phage-like particle of any one of items 1-13 or a composition of any one of items 14 - 17.
19. Method for the production of a phage-like particle of any one of items 1 - 13 or a composition of any one of items 14 - 17.
20. Use of the phage-like particle of any one of items 1 - 13 or a composition of any one of items 14 - 17 or a phage-like particle or a composition provided the system of item 18 or produced by the method of item 19, in medicine, chemistry, biotechnology, agriculture and/or food industry.

## Claims

1. Synthetic phage-like particle having specificity for at least two different target cells.

2. The synthetic phage-like particle of claim 1,
wherein said synthetic phage-like particle comprises at least a functional tail comprising a baseplate and a multi tail fiber system,
which multi tail fiber system comprises at least two different tail fibers having specificity for different target cells,
wherein said tail fibers preferably emanate from said baseplate.

3. The synthetic phage-like particle of any of claim 1 or 2 having a mass of 10⁵- 10⁷ Da.

4. The synthetic phage-like particle of any of claims 1-3,
wherein the multi-tail fiber system comprises tail fiber bundles that comprise tail fibers, preferably up to 10, more preferably four, even more preferably three tail fibers, wherein the tail fibers in a tail fiber bundle may have specificity for the same or different target cells, i.e. wherein the tail fibers are different, e.g. engineered in a different way.

5. The synthetic phage-like particle according to any one of claims 1 - 4,
wherein the tail-fiber bundles comprise at least two different types of tail-fibers having specificity to different target cells.

6. The synthetic phage-like particle of any of claims 1 - 5,
which is a synthetic headless phage or a synthetic bacteriocin preferably a synthetic tailocin, diffocin, monocin or pyocin.

7. The synthetic phage-like particle of any of claims 1-6,
wherein tail fibers are derived from bacteriophages selected from the group comprising Caudovirales (familiy of Myoviridae, Autographiviridae, Podoviridae, Ackermannviridae, and Siphoviridae), Leviviridae, Tectiviridae, Corticoviridae, Plasmaviridae, Sphaerolipoviridae, Inoviridae, Microviridae, Picobirnaviridae and/or Cystoviridae, or from bacteriocin producing bacteria selected from the group comprising Pseudomonas aeruginosa Klebsiella and/or Clostridium difficile.

8. The synthetic phage-like particle of any of claims 1-7,
wherein the target cells are selected from the group comprising bacterial cells, such as E. coli, Pseudomonas and/or Yersinia, eucaryotic cells, plant and/or mammalian cells, in particular antibiotic resistant bacterial cells selected from the group comprising methicillin-resistant Staphylococcus aureus (MRSA), Vancomycin-resistant Enterococcus (VRE), multidrug-resistant Mycobacterium tuberculosis (MDR-TB), carbapenem-resistant Enterobacteriaceae (CRE), multidrug-resistant Acinetobacter baumannii, (multi-)drug resistant Pseudomonas aeruginosa and/or Neisseria gonorrhoeae.

9. The synthetic phage-like particle of any of claims 1 - 8,
which is provided by a cell-free system, preferably by a continuous cell-free expression system.

10. The synthetic phage-like particle of any of claims 1-9,
wherein the composition and/or ratio of different tail fibers of the multi-fiber system correlates with the composition and/or ratio of nucleic acids encoding different tail fibers present during tail fiber expression.

11. Composition comprising a plurality of synthetic phage-like particles of any one of claims 1 -10.

12. The composition of any one of claim 11,
wherein the ratio of tail fiber types is adjusted and/or preselected by providing corresponding tail-fiber encoding nucleic acids in predetermined amounts and/or ratios for tail fiber expression.

13. System adapted for the production of a phage-like particle of any one of claims 1-10 or a composition of any one of claims 11 or 12.

14. Method for the production of a phage-like particle of any one of claims 1 -10 or a composition of any one of claims 11 or 12.

15. Use of the phage-like particle of any one of claims 1 - 10 or a composition of any one of claims 11 or 12 or a phage-like particle or a composition provided the system of claim 13 or produced by the method of claim 14, in medicine, chemistry, biotechnology, agriculture and/or food industry.
